(19) Europäisches Patentamt

European Patent Office

Office européen des brevets

(11) **EP 1 580 285 A1**

(12) **EUROPEAN PATENT APPLICATION**
published in accordance with Art. 158(3) EPC

(43) Date of publication:
28.09.2005 Bulletin 2005/39

(51) Int Cl.[7]: **C22C 1/02**, G06F 17/50

(21) Application number: 03809865.3

(22) Date of filing: 30.10.2003

(86) International application number:
PCT/JP2003/013928

(87) International publication number:
WO 2004/040028 (13.05.2004 Gazette 2004/20)

(84) Designated Contracting States:
AT BE BG CH CY CZ DE DK EE ES FI FR GB GR
HU IE IT LI LU MC NL PT RO SE SI SK TR

(30) Priority: 30.10.2002 JP 2002316891

(71) Applicant: National Institute for Materials Science
Ibaraki 305-0047 (JP)

(72) Inventors:
• YOKOKAWA, Tadaharu,
c/o Nat. Inst. for Mat. Sce.
Tsukuba-shi, Ibaraki 305-0047 (JP)

• HARADA, Hiroshi,
c/o Nat. Inst. for Materials Sce.
Tsukuba-shi, Ibaraki 305-0047 (JP)
• KOIZUMI, Yutaka, c/o Nat. Inst. for Materials Sce.
Tsukuba-shi, Ibaraki 305-0047 (JP)
• KOBAYASHI, Toshiharu,
c/o Nat. Inst. for Mat. Sce.
Tsukuba-shi, Ibaraki 305-0047 (JP)
• OSAWA, Makoto, c/o Nat. Inst. for Materials Sce.
Tsukuba-shi, Ibaraki 305-0047 (JP)

(74) Representative: Hall, Matthew Benjamin
Frank B. Dehn & Co.
179 Queen Victoria Street
London EC4V 4EL (GB)

(54) **GAMMA DASH PRECIPITATION STRENGTHENED PLATINUM GROUP ELEMENT-ADDED Ni-BASED SUPERALLOY DESIGNING SUPPORT PROGRAM AND GAMMA DASH PRECIPITATION STRENGTHENED PLATINUM GROUP ELEMENT-ADDED Ni-BASED SUPERALLOY DESIGNING SUPPORT APPARATUS**

(57) A novel program for supporting design of a $\gamma'$ precipitation strengthened platinum group element-added Ni-based superalloy. To support the design, a computer is allowed to function as input means (1) for inputting the composition, working temperature, and working stress of an Ni-based superalloy, storage means (2) for previously storing the constituent elements of the Ni-based superalloy, the structure factor calculation formula thereof, and the alloy characteristics calculation formula thereof, structure factor calculating means (3) for calculating the structure factor from the alloy composition by using the structure factor calculation formula read from the storage means (2), alloy characteristics calculating means (4) for calculating an alloy characteristics from the alloy composition, the structure factor, the working temperature, and the working stress by using the alloy characteristics calculate formula read out of the storage means (2), and output mean (5) for outputting the structure factor and alloy characteristics together with the alloy composition. Therefore, alloy characteristics analysis and alloy composition search of a $\gamma'$ precipitation strengthened platinum group element-added Ni-based superalloy are simply, easily, and efficiently conducted. A design supporting device therefor is also disclosed.

Fig. 1

**Description**

Technical Field

The present invention relates to a γ′ precipitation strengthened platinum group element-added Ni-based superalloy designing support program and a γ′ precipitation strengthened platinum group element-added Ni-based superalloy designing support apparatus. More particularly, the present invention relates to a novel design support program of a γ′ precipitation strengthened platinum group element-added Ni-based superalloy and its design support apparatus which are useful for alloy characteristic analysis and alloy composition search of a γ′ precipitation strengthened Ni-based superalloy with a platinum group element added.

Background Art

Heretofore, the γ′ precipitation strengthened Ni-based superalloy has been widely used as a heat resistance alloy having excellent high temperature strength in a high temperature member of a heat engine such as a jet engine and a power generation gas turbine. In particular, the γ′ precipitation strengthened Ni-based superalloy is essential to turbine blades which are used at high temperature and high pressure, and high temperate characteristics of this alloy have significant effect on the power output and thermal efficiency of the heat engine. Further, nowadays, from the viewpoint of global warming prevention and $CO_2$ saving, ultra-high efficiency is demanded in the power generation gas turbine system and it is hoped that the higher performance alloy will be developed.

The γ′ precipitation strengthened Ni-based superalloy is composed of more than ten elements such as Ni, Al, Co, Cr, Mo, W, Ti, Ta, Hf, and Re. In the fourth-generation alloy having excellent creep strength at higher temperature and long time life due to the latest advancement in microstructural stability, platinum group elements are added such as ruthenium (Ru) and iridium (Ir). In order to develop a new alloy having excellent performance, it is necessary to design and manufacture alloys of different composition combining these constituent elements and verify their performance, consuming a tremendous labor and cost. It is hence desired to optimize the ratio and composition of alloy composition phase at working temperature and stress easily and efficiently, but at the present, the technology applicable to development of γ′ precipitation strengthened Ni-based superalloy is limited only to PHACOMP (non-patent reference 1), DV-Xα cluster method (non-patent reference 2), and γ′ precipitation strengthened Ni-based superalloy designing support apparatus (patent reference 1).

However, PHACOMP is a technology which judges harmful phase on the basis of quantum theory of electrons and is not intended to develop a new alloy.

DV-Xα cluster method is a method of finding composition of a new alloy by using energy level and covalent bond degree of alloy elements as parameters, but it makes no consideration about structural factors directly relating to the alloy performance such as lattice constant misfit of composition phase and is not applicable to platinum group elements.

The γ′ precipitation strengthened Ni-based superalloy designing support apparatus is an excellent alloy design support apparatus wherein the alloy composition is inputted, and the convergent calculation of composition and partitioning ratio of γ phase which is the constituent phase and the convergent calculation of volume fraction of γ′ phase are coupled with each other, and structural factors which are composition of γ phase and γ′ phase, volume fraction of γ′ phase, and lattice misfit are calculated, and then the mechanical performance is calculated from the structural factors and alloy composition. However, the apparatus requires the structural factor calculation to be fixed at 900°C and is not applicable to platinum group elements.

Non-patent reference 1: Chester T. Sims et al., "Superalloys II," John Wiley & Sons, Inc., 1987, pp. 230 - 233.

Non-patent reference 2: Yoshiyuki Kowada, "DV-Xα home page," [online], updated October 10, 2002, DV-Xα Study Society, [searched October 28, 2002], Internet <URL: http://www.sci.hyogo-u.ac.jp/ykowada/>

Patent reference 1: Japanese Patent Application Laid-Open No. 3-191032

In the light of the above background, hence, it is an object of the present invention to present a novel γ′ precipitation strengthened platinum group element-added Ni-based superalloy designing support program and γ′ precipitation strengthened platinum group element-added Ni-based superalloy designing support apparatus capable of easily and efficiently performing the structural factor calculation at working temperature of γ′ precipitation strengthened Ni-based superalloy with a platinum group element added, the analysis of alloy characteristics such as creep resistant characteristics at working stress and further the search for a new γ′ precipitation strengthened platinum group element added Ni-based superalloy satisfying the required performance, all of which could not be realized by the above prior arts.

Disclosure of Invention

To solve the above problems, it is a first aspect of the present invention to provide a γ′ precipitation strengthened platinum group element-added Ni-based superalloy designing support program which, in order to support designing of γ′ precipitation strengthened platinum group element-added Ni-based superalloy, makes the computer to function as input means for inputting alloy composition, working temperature and working stress of Ni-based superalloy, storage means for preliminarily storing Ni-based superalloy constituent elements, structural factor formula, and alloy characteristics formula, structural factor calculating means for calculating the structural factors from the alloy composition by using the structural factor formula being read out from the storage means, alloy characteristics calculating means for calculating the alloy characteristics from the alloy composition, structural factor, working temperature, and working stress by using the alloy characteristics formula being read out from the storage means, and output means for outputting the structural factor and alloy characteristics together with the alloy composition. And, it is a second aspect to provide a γ′ precipitation strengthened platinum group element-added Ni-based superalloy designing support apparatus, being an apparatus for supporting design of γ′ precipitation strengthened platinum group element-added Ni-based superalloy, comprising input means for inputting alloy composition, working temperature and working stress of Ni-based superalloy, storage means for preliminarily storing Ni-based superalloy constituent elements, structural factor formula, and alloy characteristics formula, structural factor calculating means for calculating the structural factors from the alloy composition by using the structural factor formula being read out from the storage means, alloy characteristics calculating means for calculating the alloy characteristics from the alloy composition, structural factor, working temperature, and working stress by using the alloy characteristics formula being read out from the storage means, and output means for outputting the structural factor and alloy characteristics together with the alloy composition.

It is a third aspect to provide the γ′ precipitation strengthened platinum group element-added Ni-based superalloy designing support program or its designing support apparatus, in which constituent elements stored in the storage means are Ni, Co, Cr, Mo, W, Al, Ti, Nb, Ta, Hf, Re, Ir, Ru, Rh, Pd, and Pt, it is a fourth aspect to provide the γ′ precipitation strengthened platinum group element-added Ni-based superalloy designing support program or its designing support apparatus, in which the structural factor formula stored in the storage means includes at least the equilibrium formula of γ phase and γ′ phase at working temperature, it is a fifth aspect to provide the γ′ precipitation strengthened platinum group element-added Ni-based superalloy designing support program or its designing support apparatus, in which the equilibrium formula of γ phase and γ′ phase is composed of a formula of γ′ surface at working temperature and a formula of partitioning ratio, it is a sixth aspect to provide a γ′ precipitation strengthened platinum group element-added Ni-based superalloy designing support program or its designing support apparatus, in which the alloy characteristics formula stored in the storage means is expressed as function of alloy composition, structural factor, working temperature and working stress, it is a seventh aspect to provide the γ′ precipitation strengthened platinum group element-added Ni-based superalloy designing support program, in which the computer is further functioned as γ′ phase calculating means for calculating the composition of γ phase and γ′ phase at working temperature, and the ratio of γ′ phase, by simultaneously operating iterative convergent calculation of γ′ phase composition and distribution ratio about constituent elements, and iterative calculation of γ′ phase quantity, and it is an eighth aspect to provide the γ′ precipitation strengthened platinum group element-added Ni-based superalloy designing support apparatus, further comprising γ′ phase calculating means for calculating the composition of γ phase and γ′ phase at working temperature, and the ratio of γ′ phase, by simultaneously operating iterative convergent calculation of γ′ phase composition and partitioning ratio about constituent elements, and iterative calculation of γ′ phase quantity.

Further, it is a ninth aspect of the present invention to provide a γ′ precipitation strengthened platinum group element-added Ni-based superalloy designing support program which, in order to support designing of γ′ precipitation strengthened platinum group element-added Ni-based superalloy, makes the computer to function as input means for inputting one or more required performances, working temperature and working stress of Ni-based superalloy, storage means for preliminarily storing Ni-based superalloy constituent elements, structural factor formula, and alloy characteristics formula, alloy composition calculating means for calculating the alloy composition for satisfying the required performance, structural factor calculating means for calculating the structural factors from the alloy composition by using the structural factor formula being read out from the storage means, alloy characteristics calculating means for calculating the alloy characteristics from the alloy composition, structural factor, working temperature, and working stress by using the alloy characteristics formula being read out from the storage means, and output means for outputting the structural factor and alloy characteristics together with the alloy composition. And, it is a tenth aspect to provide a γ′ precipitation strengthened platinum group element-added Ni-based superalloy designing support apparatus, being an apparatus for supporting design of γ′ precipitation strengthened platinum group element-added Ni-based superalloy, comprising input means for inputting one or more required performances, working temperature and working stress of Ni-based superalloy, storage means for preliminarily storing Ni-based superalloy constituent elements, structural factor formula, and alloy characteristics formula, alloy composition calculating means for calculating the alloy composition for satisfying the required performance, structural factor calculating means for calculating the structural factors from the alloy composition by using the structural factor formula being read out from the storage means, alloy characteristics calculating means for calculating the alloy characteristics from the alloy composition, structural factor, working temperature, and working stress by using the alloy characteristics formula being read out from the storage means, and

output means for outputting the structural factor and alloy characteristics together with the alloy composition.

It is an eleventh aspect to provide the γ′ precipitation strengthened platinum group element-added Ni-based superalloy designing support program or its designing support apparatus, in which constituent elements stored in the storage means are Ni, Co, Cr, Mo, W, Al, Ti, Nb, Ta, Hf, Re, Ir, Ru, Rh, Pd, and Pt, it is a twelfth aspect to provide the γ′ precipitation strengthened platinum group element-added Ni-based superalloy designing support program or its designing support apparatus, in which the structural factor formula stored in the storage means includes at least the equilibrium formula of γ phase and γ′ phase at working temperature, it is a thirteenth aspect to provide the γ′ precipitation strengthened platinum group element-added Ni-based superalloy designing support program or its designing support apparatus, in which the equilibrium formula of γ phase and γ′ phase is composed of a formula of γ′ surface at working temperature and a formula of distribution ratio, it is a fourteenth aspect to provide the γ′ precipitation strengthened platinum group element-added Ni-based superalloy designing support program or its designing support apparatus, in which the required performance is one or more selected from one or both of the alloy characteristics and structural factor.

According to the present invention of the above features, with the use of the computer, structural factors such as lattice constant, lattice misfit and solid solution index of alloy elements can be calculated automatically by the preliminarily stored structural factor formula, upon inputting of alloy composition, working temperature and working stress. This structural factor formula may include, for example, an equilibrium formula of γ phase and γ′ phase at working temperature in convergent calculation, more specifically, an equilibrium formula of γ phase and γ′ phase composed of a formula of γ′ surface at working temperature and formula of distribution ratio.

Further, from the inputted alloy composition, working temperature, working stress and calculated structural factor, using the alloy characteristics formula, alloy characteristics such as creep rupture life can be also calculated automatically. This alloy characteristics formula may be expressed as a function of alloy composition, structural factor, working temperature and working stress.

Furthermore, the calculated structural factor and alloy characteristics are displayed on screen together with the inputted alloy composition, and can be referred upon designing the alloy.

In addition, regarding the preliminarily stored constituent elements of Ni-based superalloy such as Ni, Co, Cr, Mo, W, Al, Ti, Nb, Ta, Hf, Re, Ir, Ru, Rh, Pd, and Pt, by coupling the iterative convergent calculation of the γ′ phase composition and partitioning ratio and the iterative calculation of the volume fraction of γ′ phase with each other, it is also possible to automatically calculate and display the composition and amount ratio of each of the γ phase and γ′ phase at the inputted working temperature.

On the other hand, upon inputting one or more required performances, more specifically, one or more required performances selected from one or both of the alloy characteristics and structural factors, the alloy composition satisfying the required performance can be calculated automatically. More specifically, while automatically changing the composition and phase amount of the γ′ phase, the composition of each γ phase is calculated sequentially and the alloy composition is calculated automatically by combining these compositions,.

Then, the structural factor can be calculated automatically based on the calculated alloy composition by the structural factor formula, and the alloy composition can be calculated automatically based on the calculated alloy composition and structural factor and the inputted working temperature and working stress by the alloy characteristics formula, and a list of structural factor, alloy characteristics and alloy composition can be outputted by screen display and such.

As a result, it is capable of easily and efficiently performing the structural factor calculation at working temperature of γ′ precipitation strengthened platinum group element-added Ni-based superalloy and analysis of alloy characteristics such as creep resistant characteristics at working stress, and further the search for a new γ′ precipitation strengthened platinum group element-added Ni-based superalloy which satisfies the required performance.

Brief Description of Drawings

Fig. 1 is a function block diagram of an embodiment of the present invention.
Fig. 2 is a flowchart of an embodiment of the present invention.
Fig. 3 is a specific calculation flowchart of composition, volume fraction, structural factor, and alloy characteristics of γ phase and γ′ phase.
Fig. 4 is a calculation flowchart successive to Fig. 3.
Fig. 5 shows results of analysis example of an embodiment of the present invention.
Fig. 6 shows results of search example of other embodiment of the present invention.

In the drawings, the following reference numerals are used.

1    Analysis system
11    Input means
12    Storage means
13    Composition factor calculating means
14    Alloy characteristics calculating means
15    γ′ phase calculating means
16    Output means
2    Search system
21    Input means
22    Storage means
23    Alloy composition calculating means
24    Composition factor calculating means
25    Alloy characteristics calculating means
26    Output means

Best Mode for Carrying Out the Invention

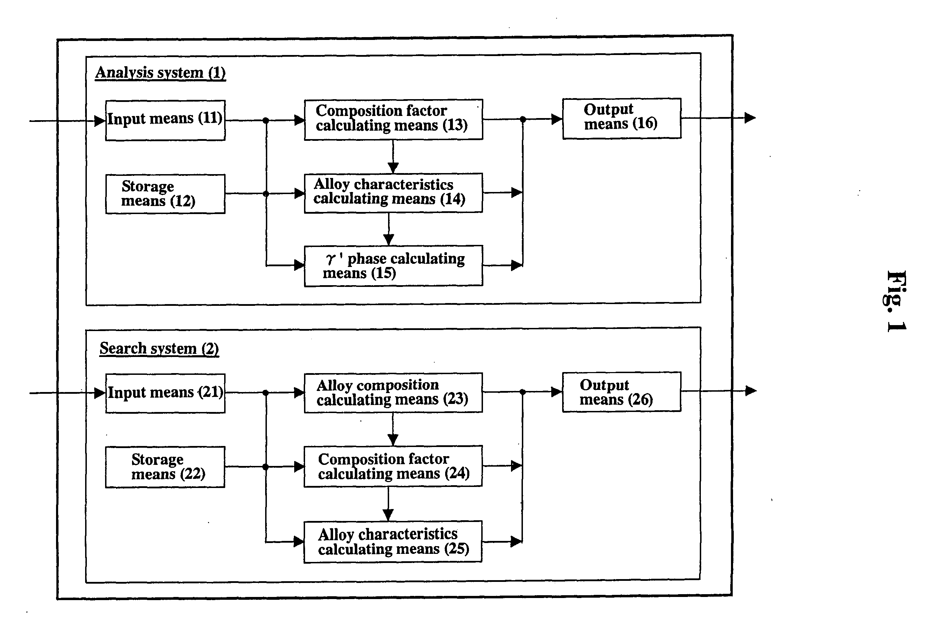

Fig. 1 to Fig. 4 are function block diagrams and flowcharts explaining an embodiment of the present invention having such features as mentioned above. The alloy characteristics analysis and alloy composition search in the embodiment are explained below.

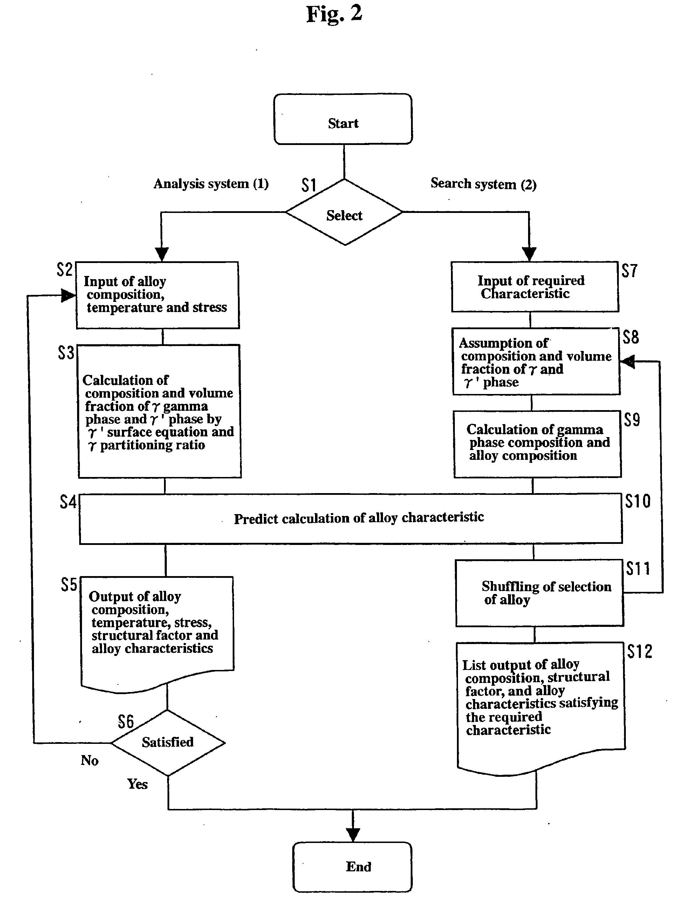

Fig. 1 and Fig. 2 show an embodiment consisting of two systems, that is, analysis system (1) for calculating and outputting alloy structural factor and alloy characteristics from the entered alloy composition, working temperature and working stress, and search system (2) for searching and outputting the alloy composition satisfying the entered required performance. In this embodiment, either the analysis system (1) or search system (2) is selected according to the purpose of use (step S1), and a desired calculation result can be obtained. In the two-system configuration, it is not required to install two units each, such as input means (11), (21), storage means (21), (22), or output means (16), (26).

<Analysis of alloy characteristic>

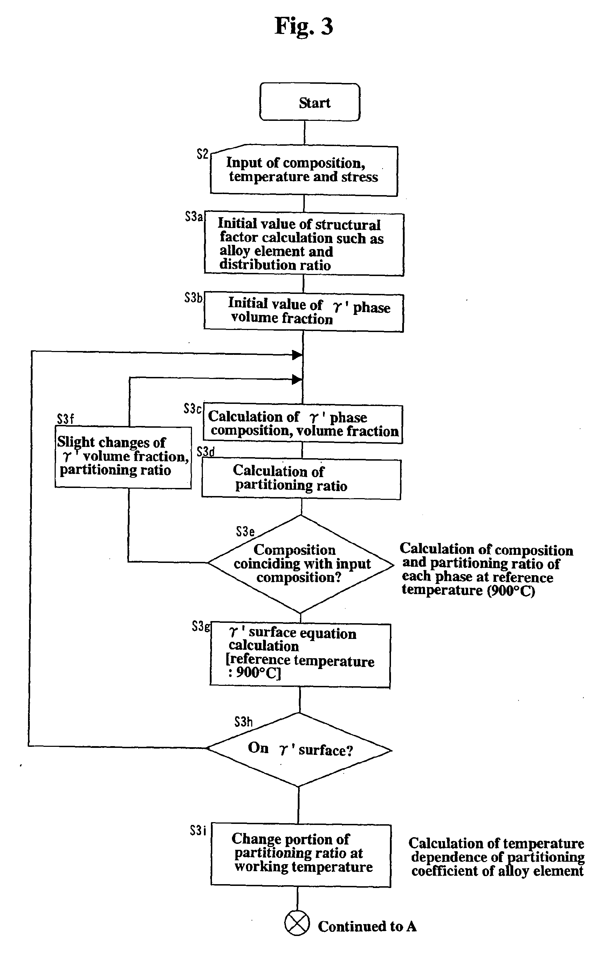

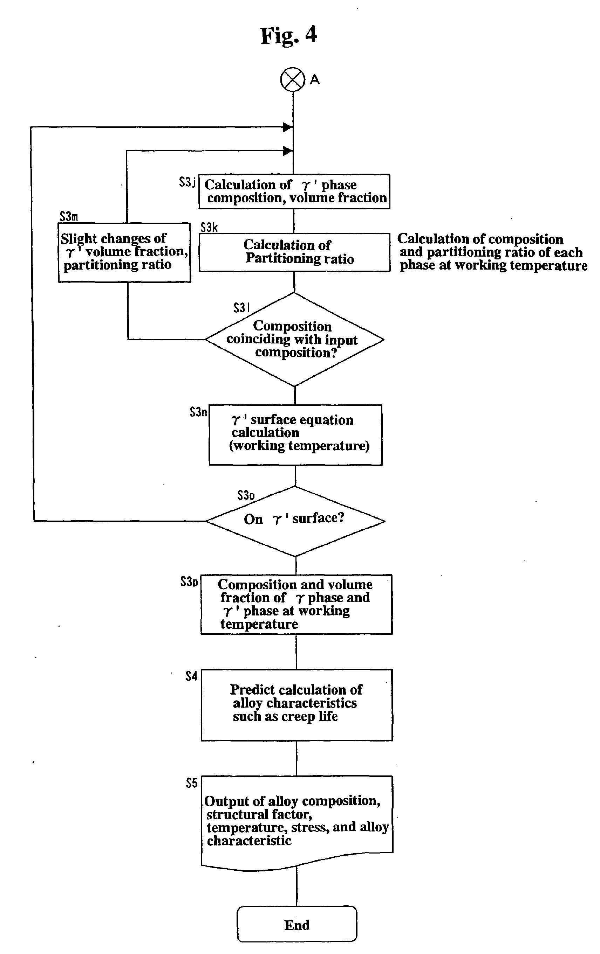

In the embodiment, by the input of alloy composition, working temperature and working stress of Ni-based superalloy of which constituent elements are Ni, Co, Cr, Mo, W, Al, Ti, Nb, Ta, Hf, Re, Ir, Ru, Rh, Pd, and Pt (Fig. 1 - input means (11), Fig. 2, Fig. 3 - step S2), the composition and volume fractions of γ phase and γ′ phase at the entered working temperature are calculated according to the equilibrium formula (Fig. 1 - composition factor calculating means (13), γ′ phase calculating means (15), Fig. 2 - step S3, Fig. 3, Fig. 4 - steps S3a to S3p). This equilibrium formula is a calculation formula of phase composition in which γ phase and γ′ phase are present in equilibrium, that is, formula of γ′ surface and formula of partitioning ratio of alloy elements (expressed by ratio of concentration of constituent elements Co, Cr, Mo, W, Al, Ti, Nb, Ta, Hf, Re, Ir, Ru, Rh, Pd, and Pt in γ phase and concentration thereof in γ′ phase). The formula of γ′ surface and formula of partitioning ratio are compiled on the basis of the analytical data of multiple regression analysis of existing Ni-based superalloy.

The formula of γ′ surface is expressed as function of constituent element concentration in γ′ phase and temperature as indicated in formula (1).

<div align="center">Al concentration in γ phase</div>

<div align="center">= f {concentration of Co, Cr, Mo, W, Al, Ti, Nb, Ta, Hf, Re, Ir, Ru, Rh, Pd, and Pt</div>

<div align="center">in γ′ phase (at%), temperature}          (1)</div>

The formula of partitioning ratio is also expressed as function of constituent element concentration in γ′ phase and temperature as indicated in formula (2).

<div align="center">Partitioning ratio of element *i*</div>

<div align="center">= g {concentration of Co, Cr, Mo, W, Al, Ti, Nb, Ta, Hf, Re, Ir, Ru, Rh, Pd, and Pt</div>

$$\text{in } \gamma' \text{ phase (at\%), temperature}\} \tag{2}$$

Herein, i is any one of Co, Cr, Mo, W, Al, Ti, Nb, Ta, Hf, Re, Ir, Ru, Rh, Pd, and Pt.

Using the formula of γ surface and formula of partitioning ratio represented by formulas (1) and (2), the iterative convergence is calculated according to the calculation flow shown in Fig. 3 and Fig. 4. In this embodiment, in order to assure stability of iterative convergent calculation and because the analytical data is abundant, first, the composition and volume fraction of γ phase and γ' phase are calculated, in principle, at 900°C (this temperature is only an example) (steps S3a to S3h). More specifically, in this calculation, by using the input values of alloy composition, working temperature, and working stress (step S2), the initial value of structural factor calculation such as partitioning ratio of alloying elements (step S3a), and initial value of fraction of γ' phase (step S3b), calculation of γ' phase composition and volume fraction (step S3c) and partitioning ratio (step S3d) is repeated (first iterative convergent calculation) until the input alloy composition and calculated alloy composition are matched (step S3e), while slightly changing the volume fraction of γ' and partitioning ratio (step S3f), and after reaching coincidence, the equation of γ' surface is calculated at reference temperature 900 degrees (step S3g), and the process (steps S3c to S3g) is repeated until the calculation result is put on the γ' surface (step S3h).

Next, calculating the differential portion of the γ' surface and partitioning ratio due to difference between the entered working temperature and the reference temperature 900°C (step S3i), the composition and volume fraction of γ phase and γ' phase at the entered working temperature are calculated (steps S3j to S3o). More specifically, in this calculation, second iterative convergent calculation similar to the first iterative convergent calculation is operated at the working temperature.

If the result of γ' surface equation calculation (step S3n) is on the γ' surface (step S3o), it means that the composition and volume fraction of γ phase and γ' phase at working temperature are calculated (step S3p).

As shown in Fig. 3 and Fig. 4, by providing two different loops for calculation, that is, first iterative convergent calculation (steps S3c to S3h) as a loop for calculating the volume fraction of γ' phase and partitioning ratio at reference temperature 900°C, and second iterative convergent calculation (steps S3j to S3o) as another loop for calculating the volume fraction of γ' phase and partitioning at working temperature, divergence of convergent calculation answers can be suppressed, and stable analysis results of high precision will be obtained.

If harmful phase is produced from the entered alloy composition or γ' phase deposits, it is so indicated. In this case, by re-inputting of alloy composition, working temperature and working stress, the composition and volume fraction of γ phase and γ' phase can be calculated again.

After calculating the composition and volume fraction of γ phase and γ' phase, other structural factors and alloy characteristics shown in Table 1 below are predicted and calculated (Fig. 1 - composition factor calculating means (13), alloy characteristics calculating means (14), γ' phase calculating means (15), Fig. 2, Fig. 4- step S4). The calculation formula of structural factor and alloy characteristics formula of alloy are calculated preliminarily (Fig. 1- storage means (12)).

Table 1

| Examples of structural factors and alloy characteristics which can be calculated | |
|---|---|
| Structural Factors | Alloy Characteristics |
| At working temperature:<br><br>    composition and volume fraction of γ phase and γ' phase;<br><br>    lattice constant of γ phase and γ' phase;<br>    lattice misfit;<br>    specific gravity (room temperature);<br>    liquidus temperature;<br>    solidus temperature;<br>    incipient melting temperature;<br>    solid solution temperature;<br>    solid solution temperature width; and<br>    solbility limit index (900°C). | at working temperature and working stress:<br>    creep rupture life of single crystal superalloy;<br><br>    elongation of single crystal superalloy; and<br>    reduction of area of single crystal superalloy. |

In the present invention, by multiple regression analysis of existing data of Ni-based superalloy, it is one of the features that the alloy characteristics formula is used as function of alloy composition and structural factor, alloy composition and working temperature, alloy composition and working stress, alloy composition, structural factor and working temperature, alloy composition, structural factor and working stress, alloy composition, working temperature and working stress, alloy composition, structural factor, working temperature and working stress. Accordingly, by calculating various alloy characteristics shown in Table 1, they can be displayed together with the entered alloy composition and calculated structural factor.

<Search for alloy composition>

When searching for composition of a new alloy having desired characteristic, first, the search system is selected (Fig. 1 - search system (2), Fig. 2 - step S1), and the working temperature, working stress, and required performance are entered (Fig. 1 - input means (21), Fig. 2 - step S7). As the required performance, any desired one or more can be selected from the alloy characteristics listed in Table 1. As required, one or more structural factors may be selected, and added as required performance.

By input of required performance, the alloy composition slightly changes automatically, and same as in the analysis system (1) mentioned above, the composition of the $\gamma$ phase balanced with the $\gamma'$ phase equivalent to the composition is calculated (Fig. 1- alloy composition calculating means (23), Fig. 2 - steps S8, S9), and the structural factor and alloy characteristics at working temperature and working stress are calculated (Fig. 1- structural factor calculating means (24), alloy characteristics calculating means (25), Fig. 2 - step 10). Comparing these values with the entered required performance, when the required performance is satisfied, by ranking as required (level ranking means, not shown), the calculated alloy composition, structural factor, and alloy characteristics are stored (calculating result storage means, not shown), and a list thereof is issued (Fig. 1 - output means (26), Fig. 2 - step S12).

If the required performance is not satisfied, on the other hand, the alloy composition is changed again (Fig. 1 - alloy composition calculating means (23), Fig. 2 - step S11), calculation of composition and volume fraction of $\gamma'$ phase is repeated (Fig. 1 - alloy composition calculating means (23), structural factor calculating means (24), alloy characteristics calculating means (25), Fig. 2 - steps S8 to S10). If there is no alloy satisfying the required performance, it is so indicated (Fig. 1 - output means (26)). This process is also done automatically throughout.

Embodiments

<Analysis embodiment>

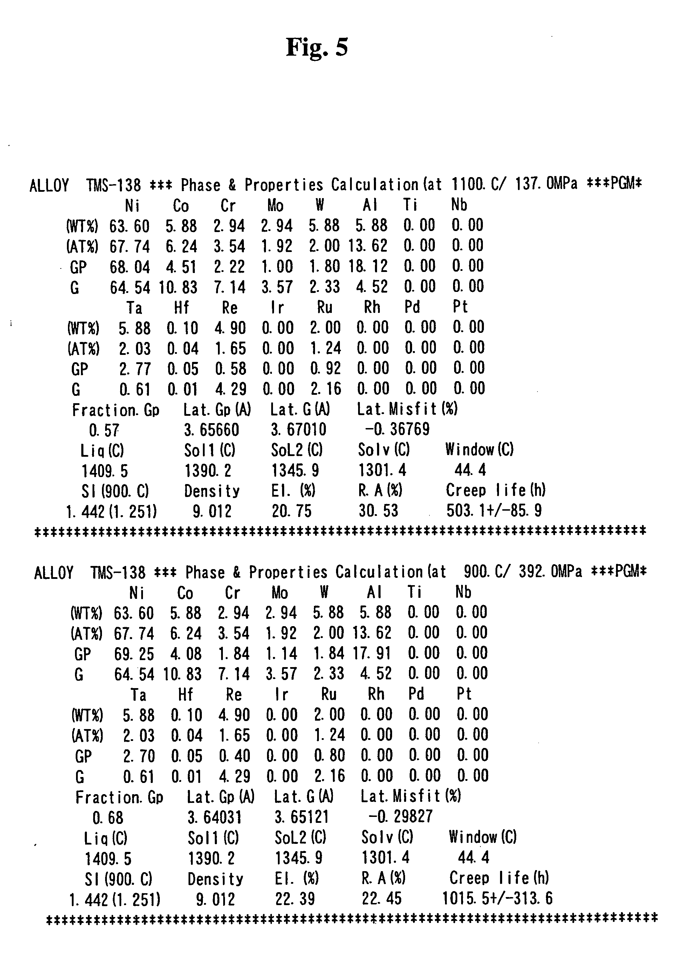

Inputting the composition of TMS-138 alloy, the equilibrium state and alloy characteristics in 900°C - 392 MPa and 1100°C - 137 MPa were calculated. Fig. 5 is an example of printed output of the results. Symbols in the diagram are defined as shown in Table 2.

Table 2

| Symbol | Structural factor or Characteristic | Unit | Unit or State |
|---|---|---|---|
| (WT%) | Alloy composition | wt % | - |
| (AT%) | Alloy composition | atom % | - |
| GP | $\gamma'$ phase composition | atom % | working temperature (°C) |
| G | $\gamma$ phase composition | atom % | working temperature (°C) |
| Fraction. GP | $\gamma'$ phase volume fraction | at amount | working temperature (°C) |
| Lat. GP | $\gamma'$ phase lattice constant | Å | working temperature (°C) |
| Lat. G | $\gamma$ phase lattice constant | Å | working temperature (°C) |
| Lat. Misfit | Lattice misfit | % | working temperature (°C) |
| Liq | Liquidus temperature | °C | - |
| Sol1 | Solidus temperature | °C | - |
| Sol2 | Incipient melting temperature | °C | - |
| Solv | Complete solid solution temperature | °C | - |
| Window | Complete solid solution temperature width | °C | - |
| SI | solubility limit index | - | 900°C |

Table 2   (continued)

| Symbol | Structural factor or Characteristic | Unit | Unit or State |
|---|---|---|---|
| Density | Specific gravity | g/cm$^2$ | room temperature (°C) |
| El. | Elongation | % | working temperature (°C) |
| R.A | Reduction of area | % | working temperature (°C) |
| Creep life | Creep rupture life of single crystal alloy | h | working temperature (°C) working stress (MPa) |

Predicted values of structural factor and alloy characteristics of composition of γ phase and γ' phase shown in the printed output in Fig. 5 coincided sufficient with the measure values of TMS-138 alloy. The calculation time was about 1 second, and it has been confirmed that the alloy characteristics can be analyzed at high speed and at high precision.

<Search embodiment>

The required characteristics were creep rupture life (test condition 1100°C, 137 MPa) of 500 hours or more, and the γ phase solid solution temperature width of 40°C or more, and also the lattice misfit and solubility index were limited by certain conditions, and the vicinity of composition range of TMS-138 alloy was searched. As a result, when Ru was increased from 2.0 wt% to 2.5 wt%, it has been found that TMS-157 alloy having alloy characteristics equivalent to that of TMS-138 alloy can be obtained if other alloying elements are decreased by the increment portion of Ru.

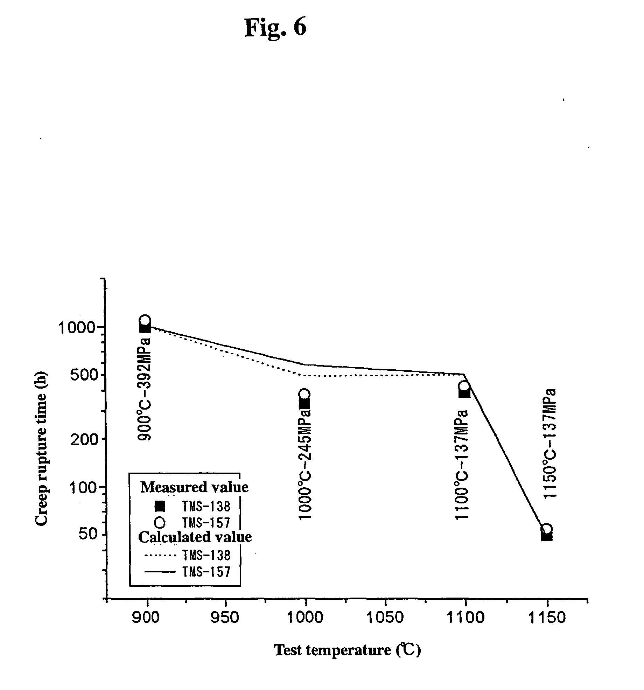

Actually, the alloy characteristics of the single crystal test piece manufactured by casting this alloy was measured, and compared with the design values. Fig. 6 shows the results. As clear from Fig. 6, design values (= calculated values) of TMS-138 and TMS-157 alloys are found to coincide sufficiently with the measured values at both high temperature and low stress side, and medium and low temperature and high stress side.

The present invention is not limited to the embodiment and example, but may be changed or modified in details.

Industrial Applicability

As described specifically herein, the present invention can predict the characteristics about arbitrary alloy composition, temperature and stress of a γ' precipitation strengthened Ni-based superalloy including a platinum group element-added superalloy. It is also possible to search efficiently a new γ' precipitation strengthened Ni-based superalloy including a new platinum group element-added superalloy satisfying the required characteristics. Hence, it is easy to analyze and search a Ni-based superalloy including a platinum group element-added superalloy, thereby significantly enhancing the efficiency of alloy developments.

**Claims**

1.   A γ' precipitation strengthened platinum group element-added Ni-based superalloy designing support program, in order to support designing of γ' precipitation strengthened platinum group element-added Ni-based superalloy, which cause the computer to function as:

　　　input means for inputting alloy composition, working temperature and working stress of Ni-based superalloy;
　　　storage means for preliminarily storing Ni-based superalloy constituent elements, structural factor formula, and alloy characteristics formula;
　　　structural factor calculating means for calculating the structural factors from the alloy composition by using the structural factor formula being read out from the storage means;
　　　alloy characteristics calculating means for calculating the alloy characteristics from the alloy composition, structural factor, working temperature, and working stress by using the alloy characteristics formula being read out from the storage means;and
　　　output means for outputting the structural factor and alloy characteristics together with the alloy composition.

2.   A γ' precipitation strengthened platinum group element-added Ni-based superalloy designing support apparatus, being an apparatus for supporting designing of γ' precipitation strengthened platinum group element-added Ni-based superalloy, comprising:

input means for inputting alloy composition, working temperature and working stress of Ni-based superalloy;

storage means for preliminarily storing Ni-based superalloy constituent elements, structural factor formula, and alloy characteristics formula;

structural factor calculating means for calculating the structural factors from the alloy composition by using the structural factor formula being read out from the storage means;

alloy characteristics calculating means for calculating the alloy characteristics from the alloy composition, structural factor, working temperature, and working stress by using the alloy characteristics formula being read out from the storage means;and

output means for outputting the structural factor and alloy characteristics together with the alloy composition.

3. The $\gamma'$ precipitation strengthened platinum group element-added Ni-based superalloy designing support program of claim 1 or $\gamma'$ precipitation strengthened platinum group element-added Ni-based superalloy designing support apparatus of claim 2, wherein constituent elements stored in the storage means are Ni, Co, Cr, Mo, W, Al, Ti, Nb, Ta, Hf, Re, Ir, Ru, Rh, Pd, and Pt.

4. The $\gamma'$ precipitation strengthened platinum group element-added Ni-based superalloy designing support program of claim 1 or $\gamma'$ precipitation strengthened platinum group element-added Ni-based superalloy designing support apparatus of claim 2, wherein the structural factor formula stored in the storage means includes at least the equilibrium formula of gamma phase and $\gamma'$ phase at working temperature.

5. The $\gamma'$ precipitation strengthened platinum group element-added Ni-based superalloy designing support program of claim 4 or $\gamma'$ precipitation strengthened platinum group element-added Ni-based superalloy designing support apparatus of claim 4, wherein the equilibrium formula of $\gamma$ phase and $\gamma'$ phase is composed of a formula of $\gamma'$ surface at working temperature and a formula of partitioning ratio.

6. The $\gamma'$ precipitation strengthened platinum group element-added Ni-based superalloy designing support program of claim 1 or $\gamma'$ precipitation strengthened platinum group element-added Ni-based superalloy designing support apparatus of claim 2, wherein the alloy characteristics formula stored in the storage means is expressed as function of alloy composition, structural factor, working temperature and working stress.

7. The $\gamma'$ precipitation strengthened platinum group element-added Ni-based superalloy designing support program of claim 1, wherein the computer is further functioned as $\gamma'$ phase calculating means for calculating the composition of $\gamma$ phase and $\gamma'$ phase at working temperature, and the amount ratio of $\gamma'$ phase, by simultaneously operating iterative convergent calculation of $\gamma'$ phase composition and partitioning ratio about constituent elements, and iterative calculation of $\gamma'$ phase quantity.

8. The $\gamma'$ precipitation strengthened platinum group element-added Ni-based superalloy designing support apparatus of claim 2, further comprising $\gamma'$ phase calculating means for calculating the composition of $\gamma$ phase and $\gamma'$ phase at working temperature, and the volume fraction of $\gamma'$ phase, by simultaneously operating iterative convergent calculation of $\gamma'$ phase composition and partitioning ratio about constituent elements, and iterative calculation of $\gamma'$ phase quantity.

9. A $\gamma'$ precipitation strengthened platinum group element-added Ni-based superalloy designing support program, in order to support designing of $\gamma'$ precipitation strengthened platinum group element-added Ni-based superalloy, which cause the computer to function as:

input means for inputting one or more required performances, working temperature and working stress of Ni-based superalloy;

storage means for preliminarily storing Ni-based superalloy constituent elements, structural factor formula, and alloy characteristics formula;

alloy composition calculating means for calculating the alloy composition for satisfying the required performance;

structural factor calculating means for calculating the structural factors from the alloy composition by using the structural factor formula being read out from the storage means;

alloy characteristics calculating means for calculating the alloy characteristics from the alloy composition, structural factor, working temperature, and working stress by using the alloy characteristics formula being read out from the storage means;and

output means for outputting the structural factor and alloy characteristics together with the alloy composition.

10. A γ′ precipitation strengthened platinum group element-added Ni-based superalloy designing support apparatus, being an apparatus for supporting design of γ′ precipitation strengthened platinum group element-added Ni-based superalloy, comprising:

> input means for inputting one or more required performances, working temperature and working stress of Ni-based superalloy;
> storage means for preliminarily storing Ni-based superalloy constituent elements, structural factor formula, and alloy characteristics formula;
> alloy composition calculating means for calculating the alloy composition for satisfying the required performance;
> structural factor calculating means for calculating the structural factors from the alloy composition by using the structural factor formula being read out from the storage means;
> alloy characteristics calculating means for calculating the alloy characteristics from the alloy composition, structural factor, working temperature, and working stress by using the alloy characteristics formula being read out from the storage means;and
> output means for outputting the structural factor and alloy characteristics together with the alloy composition.

11. The γ′ precipitation strengthened platinum group element-added Ni-based superalloy designing support program of claim 9 or γ′ precipitation strengthened platinum group element-added Ni-based superalloy designing support apparatus of claim 10, wherein constituent elements stored in the storage means are Ni, Co, Cr, Mo, W, Al, Ti, Nb, Ta, Hf, Re, Ir, Ru, Rh, Pd, and Pt.

12. The γ′ precipitation strengthened platinum group element-added Ni-based superalloy designing support program of claim 9 or γ′ precipitation strengthened platinum group element-added Ni-based superalloy designing support apparatus of claim 10, wherein the structural factor formula stored in the storage means includes at least the equilibrium formula of γ phase and γ′ phase at working temperature.

13. The γ′ precipitation strengthened platinum group element-added Ni-based superalloy designing support program of claim 12 or γ′ precipitation strengthened platinum group element-added Ni-based superalloy designing support apparatus of claim 12, wherein the equilibrium formula of γ phase and γ′ phase is composed of a formula of γ′ surface at working temperature and a formula of distribution ratio.

14. The γ′ precipitation strengthened platinum group element-added Ni-based superalloy designing support program of claim 9 or γ′ precipitation strengthened platinum group element-added Ni-based superalloy designing support apparatus of claim 10, wherein the required performance is one or more selected from one or both of the alloy characteristics and structural factor.

# Fig. 1

**Analysis system (1)**

Input means (11) → Composition factor calculating means (13) → Output means (16)

Storage means (12)

Composition factor calculating means (13) → Alloy characteristics calculating means (14) → γ' phase calculating means (15)

**Search system (2)**

Input means (21) → Alloy composition calculating means (23) → Output means (26)

Storage means (22)

Alloy composition calculating means (23) → Composition factor calculating means (24) → Alloy characteristics calculating means (25)

# Fig. 2

```
                        ┌─────────────┐
                        │    Start    │
                        └──────┬──────┘
                               │
  Analysis system (1)    S1    │         Search system (2)
              ┌───────────◇──────────────┐
              │          Select           │
              ▼                           ▼
```

**S2** Input of alloy composition, temperature and stress

**S7** Input of required Characteristic

**S3** Calculation of composition and volume fraction of $\gamma$ gamma phase and $\gamma$' phase by $\gamma$' surface equation and $\gamma$ partitioning ratio

**S8** Assumption of composition and volume fraction of $\gamma$ and $\gamma$' phase

**S9** Calculation of gamma phase composition and alloy composition

**S4** / **S10** Predict calculation of alloy characteristic

**S5** Output of alloy composition, temperature, stress, structural factor and alloy characteristics

**S11** Shuffling of selection of alloy

**S12** List output of alloy composition, structural factor, and alloy characteristics satisfying the required characteristic

**S6** Satisfied

No

Yes

```
                        ┌─────────────┐
                        │     End     │
                        └─────────────┘
```

# Fig. 3

```
                    ┌─────────────┐
                    │    Start    │
                    └─────────────┘
                           │
        S2 ┌──────────────────────────────┐
           │  Input of composition,       │
           │  temperature and stress      │
           └──────────────────────────────┘
                           │
       S3a ┌──────────────────────────────┐
           │  Initial value of structural │
           │  factor calculation such as  │
           │  alloy element and           │
           │  distribution ratio          │
           └──────────────────────────────┘
                           │
       S3b ┌──────────────────────────────┐
           │  Initial value of  γ' phase  │
           │  volume fraction             │
           └──────────────────────────────┘
                           │
       S3f                 │
   ┌──────────────┐  S3c ┌──────────────────────────────┐
   │ Slight changes│     │  Calculation of  γ' phase     │
   │ of γ' volume  │     │  composition, volume fraction │
   │ fraction,     │ S3d └──────────────────────────────┘
   │ partitioning  │     ┌──────────────────────────────┐
   │ ratio         │     │  Calculation of               │
   └──────────────┘     │  partitioning ratio           │
                         └──────────────────────────────┘
                                    │
                    S3e          ◇───────◇
                             Composition              Calculation of composition
                          coinciding with input       and partitioning ratio of
                             composition?              each phase at reference
                                ◇───────◇              temperature (900°C)
                                    │
       S3g ┌──────────────────────────────┐
           │  γ' surface equation          │
           │  calculation                  │
           │  [reference temperature       │
           │  : 900°C]                     │
           └──────────────────────────────┘
                           │
       S3h              ◇───────◇
                      On  γ' surface?
                        ◇───────◇
                           │
       S3i ┌──────────────────────────────┐    Calculation of temperature
           │  Change portion of           │    dependence of partitioning
           │  partitioning ratio at       │    coefficient of alloy element
           │  working temperature         │
           └──────────────────────────────┘
                           │
                         ⊗  Continued to A
```

## Fig. 4

⊗ A

**S3j** Calculation of $\gamma'$ phase composition, volume fraction

**S3m** Slight changes of $\gamma'$ volume fraction, partitioning ratio

**S3k** Calculation of Partitioning ratio

Calculation of composition and partitioning ratio of each phase at working temperature

**S3l** Composition coinciding with input composition?

**S3n** $\gamma'$ surface equation calculation (working temperature)

**S3o** On $\gamma'$ surface?

**S3p** Composition and volume fraction of $\gamma$ phase and $\gamma'$ phase at working temperature

**S4** Predict calculation of alloy characteristics such as creep life

**S5** Output of alloy composition, structural factor, temperature, stress, and alloy characteristic

End

14

# Fig. 5

```
ALLOY  TMS-138 *** Phase & Properties Calculation (at 1100.C/ 137.0MPa ***PGM*
          Ni    Co    Cr    Mo    W     Al    Ti    Nb
(WT%)  63.60  5.88  2.94  2.94  5.88  5.88  0.00  0.00
(AT%)  67.74  6.24  3.54  1.92  2.00 13.62  0.00  0.00
 GP    68.04  4.51  2.22  1.00  1.80 18.12  0.00  0.00
 G     64.54 10.83  7.14  3.57  2.33  4.52  0.00  0.00
          Ta    Hf    Re    Ir    Ru    Rh    Pd    Pt
(WT%)   5.88  0.10  4.90  0.00  2.00  0.00  0.00  0.00
(AT%)   2.03  0.04  1.65  0.00  1.24  0.00  0.00  0.00
 GP     2.77  0.05  0.58  0.00  0.92  0.00  0.00  0.00
 G      0.61  0.01  4.29  0.00  2.16  0.00  0.00  0.00
 Fraction. Gp    Lat. Gp (A)   Lat. G (A)   Lat. Misfit (%)
     0.57          3.65660      3.67010        -0.36769
   Liq (C)        Sol1 (C)     SoL2 (C)     Solv (C)     Window (C)
   1409.5         1390.2       1345.9       1301.4        44.4
   SI (900.C)     Density      El. (%)      R.A (%)      Creep life (h)
 1.442 (1.251)     9.012        20.75        30.53        503.1+/-85.9
*****************************************************************************

ALLOY  TMS-138 *** Phase & Properties Calculation (at  900.C/ 392.0MPa ***PGM*
          Ni    Co    Cr    Mo    W     Al    Ti    Nb
(WT%)  63.60  5.88  2.94  2.94  5.88  5.88  0.00  0.00
(AT%)  67.74  6.24  3.54  1.92  2.00 13.62  0.00  0.00
 GP    69.25  4.08  1.84  1.14  1.84 17.91  0.00  0.00
 G     64.54 10.83  7.14  3.57  2.33  4.52  0.00  0.00
          Ta    Hf    Re    Ir    Ru    Rh    Pd    Pt
(WT%)   5.88  0.10  4.90  0.00  2.00  0.00  0.00  0.00
(AT%)   2.03  0.04  1.65  0.00  1.24  0.00  0.00  0.00
 GP     2.70  0.05  0.40  0.00  0.80  0.00  0.00  0.00
 G      0.61  0.01  4.29  0.00  2.16  0.00  0.00  0.00
 Fraction. Gp    Lat. Gp (A)   Lat. G (A)   Lat. Misfit (%)
     0.68          3.64031      3.65121        -0.29827
   Liq (C)        Sol1 (C)     SoL2 (C)     Solv (C)     Window (C)
   1409.5         1390.2       1345.9       1301.4        44.4
   SI (900.C)     Density      El. (%)      R.A (%)      Creep life (h)
 1.442 (1.251)     9.012        22.39        22.45        1015.5+/-313.6
*****************************************************************************
```

# Fig. 6

<table>
<tr><td colspan="2" align="center">**INTERNATIONAL SEARCH REPORT**</td><td colspan="2">International application No.<br>PCT/JP03/13928</td></tr>
</table>

| A. CLASSIFICATION OF SUBJECT MATTER |
|---|
| Int.Cl⁷ C22C1/02, G06F17/50 |

According to International Patent Classification (IPC) or to both national classification and IPC

| B. FIELDS SEARCHED |
|---|
| Minimum documentation searched (classification system followed by classification symbols)<br>Int.Cl⁷ C22C1/02, 19/00–19/03, C22F1/10, G06F17/50 |

Documentation searched other than minimum documentation to the extent that such documents are included in the fields searched
| Jitsuyo Shinan Koho | 1922–1996 | Toroku Jitsuyo Shinan Koho | 1994–2003 |
| Kokai Jitsuyo Shinan Koho | 1971–2003 | Jitsuyo Shinan Toroku Koho | 1996–2003 |

Electronic data base consulted during the international search (name of data base and, where practicable, search terms used)
JICST

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | GB 2241358 A (NATIONAL RESEARCH INSTITUTE FOR METALS), 28 August, 1991 (28.08.91), Claims & JP 3-191032 A | 2,10 |
| Y | YOKOKAWA et al., "Ni-ki Chogokinchu no Hakkinzoku Genso no γ/γ' Sobunpai", Nippon Kinzoku Gakkaishi, September 2002, Vol.66, No.9, pages 873 to 876 | 2,10 |
| Y | KOBAYASHI et al., "Dai 4 Sedai Ni-ki Ichihoko Gyoko Chogokin no Sekkei", Nippon Kinzoku Gakkaishi, Vol.66, No.9, pages 897 to 900 | 2,10 |

| ☒ Further documents are listed in the continuation of Box C. | ☐ See patent family annex. |
|---|---|

| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
|---|---|

| Date of the actual completion of the international search<br>10 December, 2003 (10.12.03) | Date of mailing of the international search report<br>24 December, 2003 (24.12.03) |
|---|---|
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

International application No.

PCT/JP03/13928

C (Continuation).   DOCUMENTS CONSIDERED TO BE RELEVANT

| Category* | Citation of document, with indication, where appropriate, of the relevant passages | Relevant to claim No. |
|---|---|---|
| Y | EP 1184473 A2  (KABUSHIKI KAISHA TOSHIBA),<br>06 March, 2002 (06.03.02),<br>Claims<br>& JP 2002-146460 A      & US 2002-62886 A1 | 2,10 |
| Y | EP 434966 A1  (GENERAL ELECTRIC CO.),<br>03 July, 1991 (03.07.91),<br>Claims<br>& US 5151249 A1      & CA 2029539 A1<br>& JP 5-5143 A | 2,10 |

Form PCT/ISA/210 (continuation of second sheet) (July 1998)

<table>
<tr><td><strong>INTERNATIONAL SEARCH REPORT</strong></td><td>International application No.<br>PCT/JP03/13928</td></tr>
</table>

**Box I   Observations where certain claims were found unsearchable (Continuation of item 2 of first sheet)**

This international search report has not been established in respect of certain claims under Article 17(2)(a) for the following reasons:

1. [X] Claims Nos.: 1, 3 to 9, 11 to 14

   because they relate to subject matter not required to be searched by this Authority, namely:
   These Claims pertain to Computer program.

2. [ ] Claims Nos.:

   because they relate to parts of the international application that do not comply with the prescribed requirements to such an extent that no meaningful international search can be carried out, specifically:

3. [ ] Claims Nos.:

   because they are dependent claims and are not drafted in accordance with the second and third sentences of Rule 6.4(a).

**Box II   Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**   [ ]   The additional search fees were accompanied by the applicant's protest.

[ ]   No protest accompanied the payment of additional search fees.

Form PCT/ISA/210 (continuation of first sheet (1)) (July 1998)